# EUROPEAN PATENT SPECIFICATION

(11) **EP 2 031 419 B1**
(45) Date of publication and mention of the grant of the patent: **20.07.2016**
(21) Application number: 08014294.6
(22) Date of filing: 11.08.2008
(51) Int. Cl.: G01S 15/89, A61B 8/06, G01F 1/00, G01F 1/66, G01F 1/72, A61B 8/12

(54) **Ultrasound diagnostic apparatus**
Diagnostisches Ultraschallgerät
Appareil de diagnostic à ultrasons

(30) Priority: 31.08.2007 JP 2007226621
(43) Date of publication of application: 04.03.2009
(73) Proprietor: Olympus Corporation, Hachioji-shi Tokyo 192-8507 (JP)
(72) Inventor: Miyaki, Hironaka, Tokyo 192-8507 (JP)
(74) Representative: von Hellfeld, Axel

(56) References cited:
- EP-A- 0 871 043
- US-A- 5 487 389
- US-A- 5 515 857
- US-A- 5 860 928

## Description

### BACKGROUND OF THE INVENTION

### 1. Field of the Invention

The present invention relates to an ultrasound diagnostic apparatus and, more particularly, to an ultrasound diagnostic apparatus capable of calculating blood flow data on the basis of sound ray data.

### 2. Description of the Related Art

Ultrasound diagnostic apparatuses capable of acquiring a tomographic image of a living body as a subject and a blood flow image within a region-of-interest of the tomographic image by transmitting an ultrasound wave into the living body and receiving a reflected wave obtained when the ultrasound wave is reflected in a living tissue as a subject site in the living body are conventionally widely used. A tomographic image and blood flow image of a living body acquired by the ultrasound diagnostic apparatus are used, e.g., when a user such as a surgeon diagnoses an invasion depth of a lesion, observes an intraorgan state, or performs other operations.

As an example of an apparatus for acquiring a tomographic image and blood flow image of a living body as described above using an electronic scan type ultrasound transducer, an ultrasound Doppler device disclosed in Japanese Patent Application Laid-Open Publication No. 1-148244 is widely known.

If blood flow image data is generated using pieces of sound ray data acquired by an electronic scan type ultrasound transducer, a process of extracting respective pieces of data at a plurality of adjacent sample points from each piece of sound ray data and then generating blood flow image data for one pixel by grouping and averaging the pieces of data at the plurality of sample points is conventionally performed, for example.

However, since the above-described process simply averages respective pieces of data at a plurality of adjacent sample points, an S/N ratio of blood flow image data obtained after averaging is not always optimal.

US 5,860,928 discloses a method and system for smoothing color flow images in a Doppler ultrasound system. A set of original digitized ultrasound signals is received for a plurality of points on a beamline, each point having a velocity signal, a magnitude of autocorrelation signal, and a power signal associated with it. For a point along beamline selected for processing, the set of digitized ultrasound signals is submitted to digital filter, which computes a set of smoothed digitized ultrasound signals by computing an average velocity signal, an average magnitude of autocorrelation signal, and an average power signal for each point in the selected beamline and points in adjacent beamlines. Color flow information is computed from a set of original digitized ultrasound signals when any of the selected magnitudes are greater than the respective thresholds. If all of the magnitudes are below the given threshold values, the point is considered to be an anomaly, and the average values are used in place of the actual values for that point to compute color flow information. The average values may be computed from points in a three by three array, or a larger array as desired.

US 5,515,857 discloses an apparatus and method for computing a bloodstream velocity image by scanning a two-dimensional region with an ultrasonic wave. A velocity profile is formed by distributing either one of average velocities and maximum velocities of bloodstream velocities at a plurality of positions arranged along a first direction, along a second direction which is perpendicular to the first direction and extending across the blood vessel. A bloodstream value is calculated from the velocity profile and a blood vessel diameter. Thus, error caused by movement of a blood vessel accompanying pulsation can be decreased. The apparatus further has means for selecting a region of interest corresponding to a region the operator desires to render on a display.

Japanese Patent Application Laid-Open Publication No. 1-148244 makes no specific reference to a technique for solving the above-described problem. Consequently, an ultrasound diagnostic apparatus in Japanese Patent Application Laid-Open Publication No. 1-148244 suffers from, e.g., a problem that a low-quality blood flow image with noise such as dot noise uncanceled is outputted to a display section such as a monitor.

The present invention has been made in consideration of the above-described points, and has as its object to provide an ultrasound diagnostic apparatus capable of outputting a blood flow image of higher quality than ever before by improving an S/N ratio of blood flow image data generated on the basis of sound ray data.

### SUMMARY OF THE INVENTION

These problems are solved by an ultrasound diagnostic apparatus according to the claim.

### BRIEF DESCRIPTION OF THE DRAWINGS

Fig. 1 is a diagram showing an example of a configuration of a main portion of an ultrasound diagnostic system in which an ultrasound diagnostic apparatus according to the present embodiment is used;
Fig. 2 is a diagram showing an example of a detailed configuration of a color data processing section of the ultrasound diagnostic apparatus in Fig. 1;
Fig. 3 is a schematic view showing an example of processing performed in a spatial averaging circuit in Fig. 2; and
Fig. 4 is a flow chart showing a variation of the processing performed in the spatial averaging circuit in Fig. 2.

### DETAILED DESCRIPTION OF THE PREFERRED EMBODIMENTS

An embodiment of the present invention will be described below with reference to the drawings.

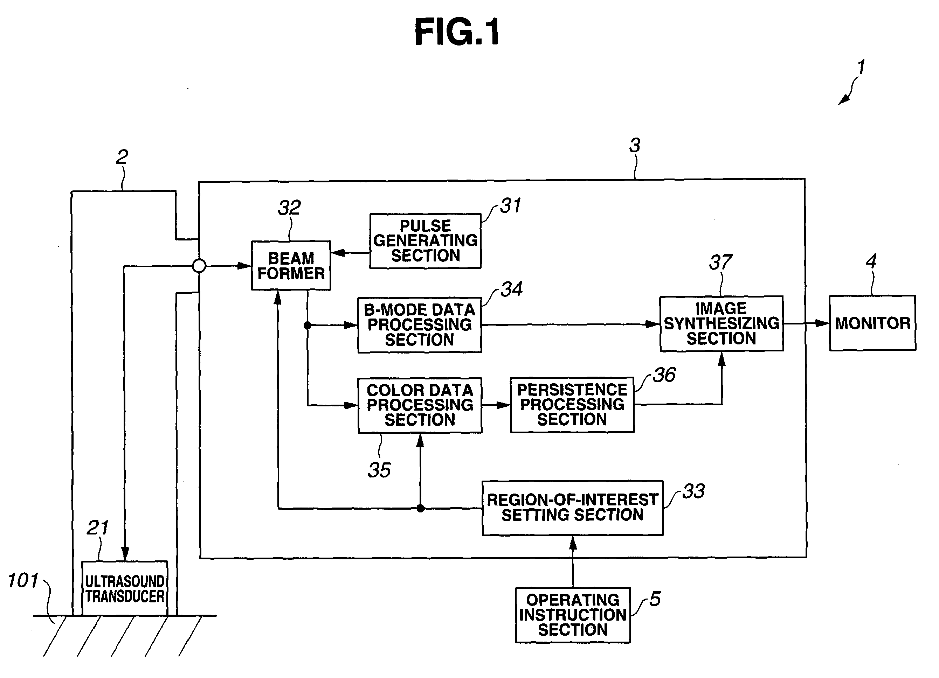

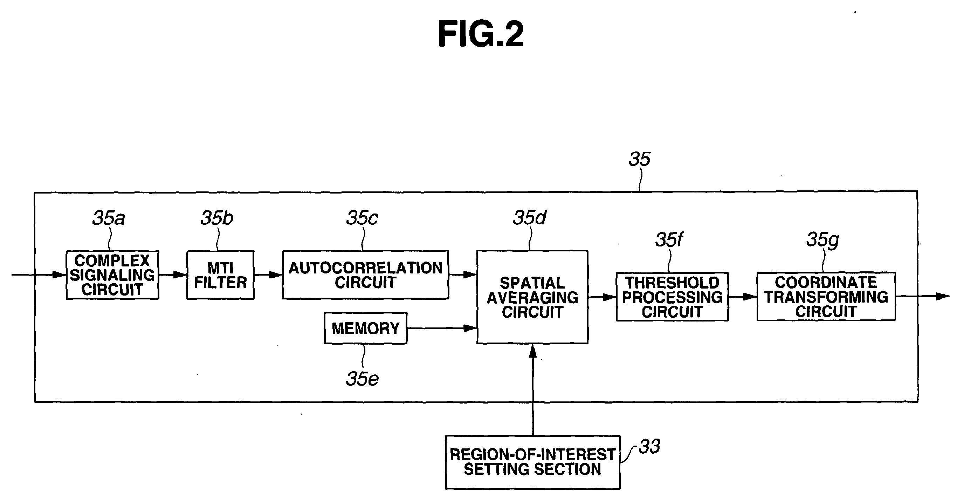

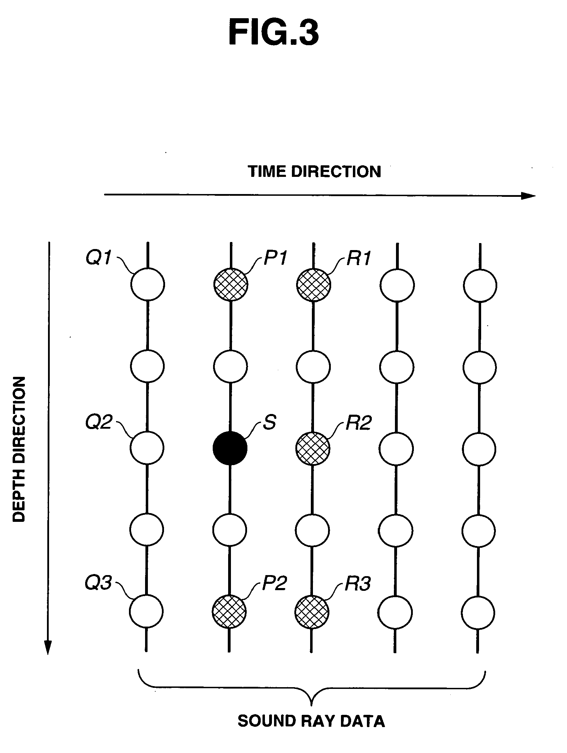

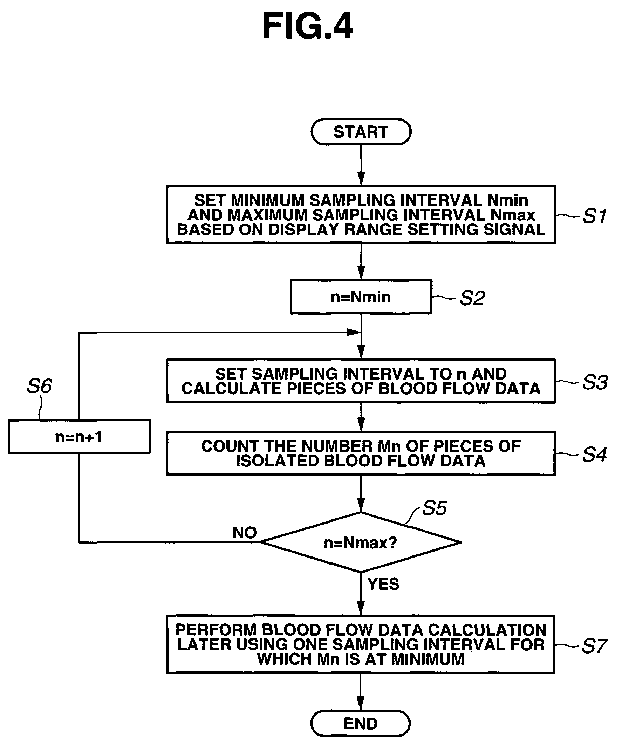

Figs. 1 to 4 relate to the embodiment of the present invention. Fig. 1 is a diagram showing an example of a configuration of a main portion of an ultrasound diagnostic system in which an ultrasound diagnostic apparatus according to the present embodiment is used. Fig. 2 is a diagram showing an example of a detailed configuration of a color data processing section of the ultrasound diagnostic apparatus in Fig. 1. Fig. 3 is a schematic view showing an example of processing performed in a spatial averaging circuit in Fig. 2. Fig. 4 is a flow chart showing a variation of the processing performed in the spatial averaging circuit in Fig. 2.

As shown in Fig. 1, a main portion of an ultrasound diagnostic system 1 is configured to have an ultrasound endoscope 2, an ultrasound diagnostic apparatus 3 which processes echo signals from the ultrasound endoscope 2 and outputs the processed echo signals as video signals, a monitor 4 which displays an image on the basis of video signals outputted from the ultrasound diagnostic apparatus 3, and an operating instruction section 5 including switches which is capable of outputting instruction signals for instructing sections of the ultrasound diagnostic system 1.

The ultrasound endoscope 2 is configured to be detachable from the ultrasound diagnostic apparatus 3 on a proximal end side and has an ultrasound transducer 21 on a distal end side.

For example, the ultrasound transducer 21 has a configuration corresponding to an electronic scan method such as linear scanning and/or convex scanning. The ultrasound transducer 21 transmits an ultrasound wave to, e.g., a subject site 101 which is a living tissue on the basis of an ultrasound signal outputted from the ultrasound diagnostic apparatus 3. The ultrasound transducer 21 receives a reflected wave of an ultrasound wave transmitted to the subject site 101 and outputs, as echo signals, respective pieces of scan information for sound rays based on the reflected wave to the ultrasound diagnostic apparatus 3.

As shown in Fig. 1, the ultrasound diagnostic apparatus 3 is configured to have a pulse generating section 31, a beam former 32, a region-of-interest setting section 33, a B-mode data processing section 34, a color data processing section 35, a persistence processing section 36, and an image synthesizing section 37. Note that the above-described sections of the ultrasound diagnostic apparatus 3 are controlled by a control section (not shown) composed of a CPU and the like.

The pulse generating section 31 outputs a pulse signal for driving the ultrasound transducer 21 to the beam former 32 on the basis of a control signal outputted from the control section (not shown).

The beam former 32 generates and outputs an ultrasound signal for generating an ultrasound wave in the ultrasound transducer 21 on the basis of a pulse signal outputted from the pulse generating section 31. The beam former 32 also performs processing such as amplification on echo signals outputted from the ultrasound transducer 21 and outputs the echo signals after the processing to the B-mode data processing section 34 and color data processing section 35. The beam former 32 further sets, by itself, range of ultrasound wave emission to the subject site 101 on the basis of a display range setting signal outputted from the region-of-interest setting section 33. The beam former 32 outputs an ultrasound signal to the ultrasound transducer 21 on the basis of the emission range set by itself.

The region-of-interest setting section 33 having a function of a region-of-interest setting section sets a display range for a blood flow image of the subject site 101 to be displayed on the monitor 4 on the basis of an instruction signal from the operating instruction section 5 and outputs, as a display range setting signal, the settings to the color data processing section 35.

The region-of-interest setting section 33 also sets a display range for a tomographic image acquired together with a blood flow image of the subject site 101 to a maximum displayable range (for the monitor 4). Additionally, the region-of-interest setting section 33 combines the settings as the display range for a tomographic image with the settings as the display range for a blood flow image and outputs, as a display range setting signal, the combination to the beam former 32.

The B-mode data processing section 34 generates a tomographic image of the subject site 101 on the basis of echo signals outputted from the beam former 32 and then outputs, as tomographic image signals, the tomographic image to the image synthesizing section 37.

The color data processing section 35 generates a piece of blood flow data which is blood flow image data within a range set by the region-of-interest setting section 33 using color Doppler processing or power Doppler processing on the basis of echo signals outputted from the beam former 32 and a display range setting signal outputted from the region-of-interest setting section 33 and outputs the generated piece of blood flow data to the persistence processing section 36.

As a configuration capable of at least one of color Doppler processing and power Doppler processing, the color data processing section 35 is configured to have a complex signaling circuit 35a, an MTI (Moving Target Indicator) filter 35b, an autocorrelation circuit 35c, a spatial averaging circuit 35d, a memory 35e storing predetermined information on spatial averaging performed in the spatial averaging circuit 35d, a threshold processing circuit 35f, and a coordinate transforming circuit 35g, as shown in, e.g., Fig. 2.

The complex signaling circuit 35a performs quadrature detection processing on echo signals outputted from the beam former 32 and outputs the resultant signals to the MTI filter 35b.

The MTI filter 35b performs filtering processing on echo signals outputted from the complex signaling circuit 35a for removing a component in slow motion in a living body and outputs the echo signals to the autocorrelation circuit 35c.

The autocorrelation circuit 35c generates a piece of complex data representing a complex autocorrelation vector for each sound ray on the basis of echo signals outputted from the MTI filter 35b and outputs, as pieces of sound ray data, the pieces of complex data to the spatial averaging circuit 35d.

The spatial averaging circuit 35d reads, from the memory 35e, predetermined information indicating a correlation between a display range set by the region-of-interest setting section 33 and a sampling interval for pieces of sound ray data outputted from the autocorrelation circuit 35c, on the basis of a display range setting signal outputted from the region-of-interest setting section 33. The spatial averaging circuit 35d then calculates, by spatial averaging, velocity and intensity of a blood flow within the range set by the region-of-interest setting section 33, on the basis of the pieces of sound ray data outputted from the autocorrelation circuit 35c and the predetermined information read from the memory 35e and outputs, as a piece of blood flow data, the calculated velocity and intensity of the blood flow to the threshold processing circuit 35f.

The threshold processing circuit 35f performs a process of removing, e.g., one whose velocity component is not more than a predetermined value and one whose intensity component falls outside a predetermined range from pieces of blood flow data outputted from the spatial averaging circuit 35d and outputs pieces of blood flow data left after the process to the coordinate transforming circuit 35g.

The coordinate transforming circuit 35g sets a coordinate position for a piece of blood flow data outputted from the threshold processing circuit 35f to be within a range set by the region-of-interest setting section 33, on the basis of the outputted piece of blood flow data and outputs the piece of blood flow data after the setting to the persistence processing section 36.

Note that the color data processing section 35 may have a configuration supporting both of a mode of performing color Doppler processing (hereinafter abbreviated as "the color Doppler mode") and a mode of performing power Doppler processing (hereinafter abbreviated as "the power Doppler mode") and may be configured to be capable of switching between the two modes on the basis of an instruction signal from the operating instruction section 5.

If the color data processing section 35 has a configuration capable of switching between the two modes, the pulse generating section 31 and beam former 32 may perform control in conjunction with switching between the color Doppler mode and the power Doppler mode such that the number of burst waves or the number of transmission of an ultrasound wave outputted from the ultrasound transducer 21 in the power Doppler mode is made larger than the number in the color Doppler mode. If the pulse generating section 31, beam former 32, and color data processing section 35 are configured in such a manner, the ultrasound diagnostic apparatus 3 can improve an S/N ratio of a blood flow image of the subject site 101 in the power Doppler mode.

The persistence processing section 36 is configured to have, e.g., table data for appropriately changing and outputting a luminance value of an inputted piece of blood flow data such that a blood flow image within a range set by the region-of-interest setting section 33 is displayed with a persistence on the monitor 4. The persistence processing section 36 performs data conversion processing on a piece of blood flow data outputted from the color data processing section 35 using the table data and outputs the piece of blood flow data after the data conversion to the image synthesizing section 37.

The image synthesizing section 37 generates video signals by combining tomographic image signals outputted from the B-mode data processing section 34 and pieces of blood flow data outputted from the persistence processing section 36 and outputs the generated video signals to the monitor 4.

Action of the ultrasound diagnostic system 1 will now be described.

First, a user inserts the ultrasound endoscope 2 into a living body or the like and brings a distal end portion of the ultrasound endoscope 2 into contact with a desired observation part of the subject site 101 in the living body. After that, the user specifies a display range for a tomographic image of the subject site 101 to be displayed on the monitor 4 by manipulating a predetermined switch or the like provided at the operating instruction section 5 and vibrates the ultrasound transducer 21 provided at the distal end portion of the ultrasound endoscope 2 to transmit an ultrasound wave to the desired observation part of the subject site 101.

The region-of-interest setting section 33 sets respective display ranges for a blood flow image and tomographic image of the subject site 101 to be displayed on the monitor 4 on the basis of an instruction signal from the operating instruction section 5 and outputs, as a display range setting signal, the settings to the beam former 32 and color data processing section 35.

The beam former 32 sets, by itself, range of ultrasound wave emission to the subject site 101 on the basis of the display range setting signal outputted from the region-of-interest setting section 33 and outputs an ultrasound signal to the ultrasound transducer 21 on the basis of the emission range set by itself.

The ultrasound transducer 21 transmits an ultrasound wave to the subject site 101 in response to the ultrasound signal outputted from the beam former 32. After that, the ultrasound transducer 21 receives a reflected wave of the ultrasound wave from the subject site 101 and outputs, as echo signals, the reflected wave to the ultrasound diagnostic apparatus 3.

The echo signals inputted to the ultrasound diagnostic apparatus 3 are outputted to the B-mode data processing section 34 and color data processing section 35 through the beam former 32.

The B-mode data processing section 34 generates a tomographic image of the subject site 101 on the basis of the echo signals outputted from the beam former 32 and then outputs, as tomographic image signals, the tomographic image to the image synthesizing section 37.

In the meantime, the echo signals inputted to the color data processing section 35 are subjected to quadrature detection processing by the complex signaling circuit 35a, are subjected to filtering processing by the MTI filter 35b, and are converted into pieces of sound ray data by the autocorrelation circuit 35c. After that, the pieces of sound ray data are outputted to the spatial averaging circuit 35d.

The spatial averaging circuit 35d calculates pieces of blood flow data within the range set by the region-of-interest setting section 33 by spatial averaging, on the basis of the pieces of sound ray data outputted from the autocorrelation circuit 35c, the display range setting signal outputted from the region-of-interest setting section 33, and predetermined information read from the memory 35e.

The spatial averaging performed by the spatial averaging circuit 35d will be specifically described.

Each point having a piece of data for one pixel (each point having a piece of data serving as a candidate for sampling) in a piece of sound ray data outputted from the autocorrelation circuit 35c is indicated by, e.g., a circle shown in Fig. 3.

The spatial averaging circuit 35d performs sampling of sound ray data outputted from the autocorrelation circuit 35c at six points in Fig. 3 (a point S, a point P1, a point P2, a point R1, a point R2, and a point R3) as sample points on the basis of the predetermined information read from the memory 35e and performs spatial averaging (signal averaging) on a piece of data at each sample point, thereby calculating a piece of blood flow data at the point S.

In other words, the spatial averaging circuit 35d having a function of a data acquiring section samples the respective pieces of data at the six points in total composed of the point S itself, the points P1 and P2, which are upwardly and downwardly spaced apart from the point S by one pixel in a depth direction of a piece of sound ray data to which the point S belongs, and the points R1, R2, and R3, which are respectively located at same depth positions as the points P1, S, and P2 in a next piece of sound ray data which is temporally continuous with the piece of sound ray data to which the point S belongs, at the time of calculating the piece of blood flow data at the point S in Fig. 3. The spatial averaging circuit 35d having a function of a blood flow data calculating section performs spatial averaging (signal averaging) on the pieces of data at the six sample points, thereby calculating the piece of blood flow data at the point S.

If sampling is performed at sample points adjacent to each other in a depth direction of a piece of sound ray data in generation of a piece of blood flow data, noise components become almost in phase with each other, and a piece of blood flow data from which noise is not adequately removed may be generated. On the other hand, if sampling is performed at sample points widely spaced apart from each other in a depth direction of a piece of sound ray data, a piece of blood flow data including artifacts may be generated. For this reason, control information such as a program for causing the spatial averaging circuit 35d to perform sampling of sound ray data while changing a sampling interval depending on a distance in a depth direction of a region-of-interest set by the region-of-interest setting section 33 is stored in advance in the memory 35e as predetermined information which optimizes an S/N ratio of a piece of blood flow data calculated in the spatial averaging circuit 35d.

Using this information, the spatial averaging circuit 35d calculates the piece of blood flow data at the point S while appropriately changing sample points as sampling sites corresponding to the points P1 and P2 in Fig. 3 to, e.g., positions upwardly and downwardly spaced apart from the point S by two pixels or positions upwardly and downwardly spaced apart from the point S by three pixels in the depth direction of the piece of sound ray data to which the point S belongs, depending on a display range set by the region-of-interest setting section 33. Note that along with a change in positions in the depth direction of the sample points corresponding to the points P1 and P2 in Fig. 3, positions in a depth direction of sample points corresponding to the points R1 and R3 in Fig. 3 are similarly changed.

Note that the spatial averaging circuit 35d does not necessarily perform sampling at the six sample points described above to calculate the piece of blood flow data at the point S in Fig. 3. More specifically, the spatial averaging circuit 35d may calculate the piece of blood flow data at the point S by, e.g., sampling pieces of data at nine sample points in total composed of points Q1, Q2, and Q3 which are respectively located at same depth positions as the points P1, S, and P2 in a previous piece of sound ray data which is temporally continuous with the piece of sound ray data to which the point S belongs and the above-described six sample points and performing spatial averaging (signal averaging) on the pieces of data at the nine sample points. The present embodiment is not limited to sampling at three sample points in one piece of sound ray data. For example, sampling may be performed at at least two sample points having a piece of data at one position included in one piece of sound ray data and a piece of data at at least one position upwardly or downwardly spaced apart from the one position by a predetermined number of pixels.

One whose velocity component is not more than a predetermined value and one whose intensity component falls outside a predetermined range are removed from the pieces of blood flow data outputted from the spatial averaging circuit 35d by the threshold processing circuit 35f. Pieces of blood flow data left after the removal are subjected to coordinate position setting by the coordinate transforming circuit 35g and are then outputted to the persistence processing section 36.

The pieces of blood flow data outputted from the color data processing section 35 are subjected to data conversion processing by the persistence processing section 36, are combined with the tomographic image signals in the image synthesizing section 37, and then are outputted to the monitor 4. With this operation, a high-quality blood flow image with noise such as dot noise suppressed is outputted to the monitor 4.

Note that an interval of sampling by the spatial averaging circuit 35d is not limited to the above-described one which is appropriately changed depending on a distance in a depth direction of the range set by the region-of-interest setting section 33. For example, the sampling interval may be a desired user-selectable sampling interval or may be a fixed sampling interval.

The ultrasound diagnostic system 1 of the present embodiment may be configured such that the sections perform operations corresponding to action to be described below, in order to achieve almost the same advantages as described above. Note that a following description will be given while a description of same portions as described above being appropriately omitted, for descriptive simplicity.

First, a user inserts the ultrasound endoscope 2 into a living body or the like and brings the distal end portion of the ultrasound endoscope 2 into contact with a desired observation part of the subject site 101 in the living body. After that, the user specifies a display range for a tomographic image of the subject site 101 to be displayed on the monitor 4 by manipulating a predetermined switch or the like provided at the operating instruction section 5.

The region-of-interest setting section 33 sets respective display ranges for a blood flow image and tomographic image of the subject site 101 to be displayed on the monitor 4 on the basis of an instruction signal from the operating instruction section 5 and outputs, as a display range setting signal, the settings to the beam former 32 and color data processing section 35.

The spatial averaging circuit 35d sets a minimum sampling interval Nmin and a maximum sampling interval Nmax in a depth direction of sound ray data on the basis of the display range setting signal outputted from the region-of-interest setting section 33 (step S1 in Fig. 4) and sets a variable n representing a sampling interval to Nmin (step S2 in Fig. 4).

After that, when an instruction to transmit an ultrasound wave to the desired observation part is given at the operating instruction section 5, the ultrasound transducer 21 transmits an ultrasound wave to the subject site 101 under control of the beam former 32. After that, the ultrasound transducer 21 receives a reflected wave of the ultrasound wave from the subject site 101 and outputs, as echo signals, the reflected wave to the beam former 32.

The echo signals inputted to the ultrasound diagnostic apparatus 3 are outputted to the B-mode data processing section 34 and color data processing section 35 through the beam former 32.

The B-mode data processing section 34 generates a tomographic image of the subject site 101 on the basis of the echo signals outputted from the beam former 32 and then outputs, as tomographic image signals, the tomographic image to the image synthesizing section 37.

In the meantime, the echo signals inputted to the color data processing section 35 are subjected to quadrature detection processing by the complex signaling circuit 35a, are subjected to filtering processing by the MTI filter 35b, and are converted into pieces of sound ray data by the autocorrelation circuit 35c. After that, the pieces of sound ray data are outputted to the spatial averaging circuit 35d.

The spatial averaging circuit 35d performs the above-described spatial averaging (signal averaging) with a sampling interval for sample points in the depth direction of sound ray data set to n pixels, on the basis of the display range setting signal outputted from the region-of-interest setting section 33 and the pieces of sound ray data outputted from the autocorrelation circuit 35c, thereby calculating pieces of blood flow data within the range set by the region-of-interest setting section 33 (step S3 in Fig. 4).

The spatial averaging circuit 35d further counts the number Mn of pieces of isolated blood flow data of the calculated pieces of blood flow data (step S4 in Fig. 4) and temporarily stores, in itself, a count result. Note that, for example, the spatial averaging circuit 35d refers to pieces of data for eight pixels adjacent to one pixel having a piece of blood flow data and extracts the piece of blood flow data at the one pixel as a piece of isolated blood flow data if all of the pieces of data for the eight pixels have no piece of blood flow data.

After that, the spatial averaging circuit 35d determines whether the variable n is equal to Nmax (step S5 in Fig. 4). If the spatial averaging circuit 35d has detected that the variable n is not equal to Nmax, the spatial averaging circuit 35d repeats the processes in steps S3 to S5 in Fig. 4 on the pieces of data outputted to the autocorrelation circuit 35c while adding 1 to the variable n (step S6 in Fig. 4), until the variable n becomes equal to Nmax. On the other hand, if the spatial averaging circuit 35d has detected that the variable n is equal to Nmax, the spatial averaging circuit 35d refers to values for Mn stored in itself. The spatial averaging circuit 35d performs blood flow data calculation later using one sampling interval for which Mn has a minimum value (step S7 in Fig. 4).

The ultrasound diagnostic system 1 of the present embodiment can output a high-quality blood flow image with noise such as dot noise suppressed to the monitor 4 even if an operation corresponding to the above-described action. Note that the present invention is not limited to the above-described embodiment and that various modifications and applications can, of course, be made.

## Claims

1. An ultrasound diagnostic apparatus (3) which generates a tomographic image of a subject site (101) in a living body by receiving as sound ray data a reflected wave of an ultrasound wave transmitted to the subject site (101) and performing predetermined signal processing on the sound ray data, comprising:
a region-of-interest setting section (33) which sets a region-of-interest in the tomographic image;
a data acquiring section (35d) which samples, from one piece of sound ray data within the region-of-interest, pieces of pixel data in at least two positions while switching between sampling intervals in order, starting with a minimum sampling interval and ending with a maximum sampling interval, in a depth direction of the sound ray data; and
a blood flow data calculating section (35, 35 a-g) which calculates a plurality of pieces of blood flow data for each of the sampling intervals by performing spatial averaging on the pieces of pixel data sampled by the data acquiring section (35d), wherein
the blood flow data calculating section (35, 35 a-g) sets, on the basis of the pieces of blood flow data for the sampling intervals, the sampling interval for which the number of isolated blood flow data is at a minimum as the sampling interval at the time of calculation of blood flow data in the sound ray data.

## Patentansprüche

1. Ultraschall-Diagnosegerät (3), das ein tomografisches Bild eines Subjektorts (101) in einem lebenden Körper erzeugt, indem es eine reflektierte Welle einer an den Subjektort (101) übertragenen Ultraschallwelle als Schallstrahldaten empfängt und an den Schallstrahldaten eine vorbestimmte Signalverarbeitung durchführt, und das umfasst:
einen Abschnitt (33) zum Festlegen eines interessierenden Bereichs, der einen interessierenden Bereich in dem tomographischen Bild festlegt;
einen Datenaufnahmeabschnitt (35d), der von einem Teil von Schallstrahldaten innerhalb des interessierenden Bereichs Teile von Pixeldaten in mindestens zwei Positionen abtastet, während er zwischen Abtastintervallen, beginnend mit einem minimalen Abtastintervall und endend mit einem maximalen Abtastintervall, in einer Tiefenrichtung der Schallstrahldaten umschaltet; und
einen Blutflussdatenberechnungsabschnitt (35, 35 a-g), der eine Mehrzahl von Teilen von Blutflussdaten für jedes der Abtastintervalle berechnet, indem er eine räumliche Durchschnittsbildung an den Teilen der durch den Datenaufnahmeabschnitt (35d) abgetasteten Teilen von Pixeldaten durchführt, wobei
der Blutflussdatenberechnungsabschnitt (35, 35 a-g) auf der Basis der Teile von Blutflussdaten für die Abtastintervalle das Abtastintervall, für das die Anzahl von isolierten Blutflussdaten bei einem Minimum liegt, als das Abtastintervall zum Zeitpunkt der Berechnung der Blutflussdaten in den Schallstrahldaten festlegt.

## Revendications

1. Appareil (3) de diagnostic par ultrasons qui génère une image tomographique d'un site sujet (101) dans un corps vivant en recevant comme données de rayons acoustiques une onde réfléchie d'une onde ultrasonore transmise jusqu'au site sujet (101) et en exécutant un traitement de signaux prédéterminé sur les données de rayons acoustiques, comprenant :
une section (33) de fixation de région d'intérêt qui fixe une région d'intérêt dans l'image tomographique ;
une section (35d) d'acquisition de données qui échantillonne, à partir d'un ensemble de données de rayons acoustiques à l'intérieur de la région d'intérêt, des ensembles de données de pixels dans au moins deux positions tout en commutant entre des intervalles d'échantillonnage, dans l'ordre, en commençant avec un intervalle d'échantillonnage minimum et en finissant avec un intervalle d'échantillonnage maximum, dans un sens de la profondeur des données de rayons acoustiques ; et
une section (35, 35a-g) de calcul de données de flux sanguin qui calcule une pluralité d'ensembles de données de flux sanguin pour chacun des intervalles d'échantillonnage en calculant une moyenne spatiale sur les ensembles de données de pixels échantillonnées par la section (35d) d'acquisition de données,
dans lequel la section (35, 35a-g) de calcul de données de flux sanguin fixe, sur la base des ensembles de données de flux sanguin pour les intervalles d'échantillonnage, l'intervalle d'échantillonnage pour lequel le nombre de données de flux sanguin isolées est à un minimum comme l'intervalle d'échantillonnage au moment du calcul de données de flux sanguin dans les données de rayons acoustiques.
